# EUROPEAN PATENT APPLICATION

(11) **EP 3 995 479 A1**
(43) Date of publication of application: **11.05.2022**
(21) Application number: 20834958.9
(22) Date of filing: 30.06.2020
(51) Int. Cl.: C07C 17/04, C07C 19/10, C09K 5/04

(54) **ALKANE PRODUCTION METHOD**

(30) Priority: 01.07.2019 JP 2019122745
(71) Applicant: Daikin Industries, Ltd., Osaka-shi, Osaka 530-8323 (JP)
(72) Inventor: ETOU, Yuusuke, Osaka-shi, Osaka 530-8323 (JP); NAKAMURA, Shingo, Osaka-shi, Osaka 530-8323 (JP)
(74) Representative: Hoffmann Eitle
(86) International application number: PCT/JP2020/025616
(87) International publication number: WO 2021/002343

(57) **Abstract**

An object of the present disclosure is to provide a chlorinated alkane at a high conversion rate (yield) and with high selectivity. Provided is a method for producing an alkane, the method including the step of subjecting an alkene to a chlorination reaction, the chlorination reaction step being performed by light irradiation using light having a wavelength in the visible light range.

## Description

### Technical Field

The present disclosure relates to a method for producing an alkane.

### Background Art

Patent Literature (PTL) 1 discloses a method for producing an alkane (2,3-dichloro-1,1,1,4,4,4-hexafluorobutane, HCFC-336mdd) by a chlorination reaction, the method comprising subjecting an alkene ((E)-1,1,1,4,4,4-hexafluoro-2-butene, E-1336mzz) to light irradiation using a mercury (Hg) arc-lamp bulb to perform the chlorination reaction.

### Citation List

### Patent Literature

PTL 1: WO2015/142981A1

### Summary of Invention

### Technical Problem

An object of the present disclosure is to produce a chlorinated alkane at a high conversion rate (yield) and a high selectivity.

### Solution to Problem

The present disclosure includes the following subject matter.

### Item 1.

A method for producing an alkane represented by formula (1): 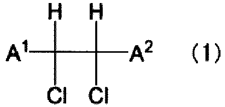 (wherein A¹ and A² are the same or different and represent a fluorine atom or a perfluoroalkyl group with the proviso that the case in which A¹ and A² both represent a fluorine atom is excluded),
the method comprising the step of subjecting an alkene represented by formula (2): 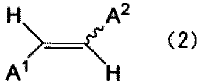 (wherein A¹ and A² are as defined above) to a chlorination reaction,
the compound of formula (2) representing a cis isomer and/or a trans isomer, and
the chlorination reaction step being performed by light irradiation using light having a wavelength in the visible light range.

### Item 2.

The production method according to Item 1, wherein the chlorination reaction is performed in a liquid phase.

### Item 3.

The production method according to Item 1 or 2, wherein the light irradiation is performed by irradiation using visible light having a wavelength of 380 nm or more and 830 nm or less.

Item 4. A composition comprising
an alkane represented by formula (1): 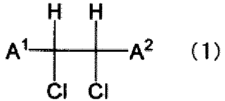 (wherein A¹ and A² are the same or different and represent a fluorine atom or a perfluoroalkyl group with the proviso that the case in which A¹ and A² both represent a fluorine atom is excluded); and
at least one additional compound comprising (selected from the group consisting of) a hydrochlorofluorocarbon (hydrochlorofluorocarbons) (HCFC) compound containing at least one chlorine (excluding the alkane represented by formula (1)).

### Item 5.

The composition according to Item 4, wherein the alkane represented by formula (1) is present in an amount of 90 mol% or more and the additional compound is present in an amount of 10 mol% or less, based on the total amount of the composition taken as 100 mol%.

### Item 6.

The composition according to Item 4 or 5, wherein the additional compound is at least one member selected from the group consisting of monochlorohexafluorobutane, trichlorohexafluorobutane, and tetrachlorohexafluorobutane.

### Item 7.

The composition according to any one of Items 4 to 6, wherein the composition is for use as an etching gas, a refrigerant, or a heat transfer medium.

### Advantageous Effects of Invention

The present disclosure enables the production of a chlorinated alkane at a high conversion rate (yield) and with a high selectivity.

### Description of Embodiments

The present inventors conducted extensive research and found that an alkane represented by formula (1) can be produced at a high conversion rate (yield) and with a high selectivity by subjecting an alkene as a starting material compound to light irradiation using light having a wavelength in the visible light range to thereby perform a chlorination reaction step.

The present inventors conducted further research on the basis of this finding and completed the present disclosure.

The present disclosure includes the following embodiments.

The method for producing an alkane represented by formula (1): 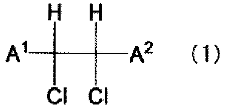 (wherein A¹ and A² are the same or different and represent a fluorine atom or a perfluoroalkyl group with the proviso that the case in which A¹ and A² both represent a fluorine atom is excluded),
the method comprising the step of subjecting an alkene represented by formula (2): 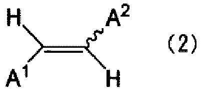
(wherein A¹ and A² are as defined above) to a chlorination reaction,
the chlorination reaction step being performed by light irradiation using light having a wavelength in the visible light range.

According to a preferred embodiment of the present disclosure, the chlorination reaction is performed in a liquid phase.

According to a preferred embodiment of the present disclosure, the light irradiation is performed by irradiation using visible light having a wavelength of 380 nm or more and 830 nm or less.

In the present disclosure, a chlorinated alkane can be produced at a high conversion rate (yield) and with a high selectivity by satisfying the requirements described above.

In the present disclosure, the "conversion rate" refers to the percentage (mol%) of the total molar amount of the compounds (e.g., chlorinated alkane etc.) other than the starting material compound (alkene) present in a gas flowing out from a reactor outlet relative to the molar amount of the starting material compound supplied to a reactor.

In the present disclosure, "selectivity" refers to the percentage (mol%) of the total molar amount of the target compound (chlorinated alkane) present in a gas flowing out from a reactor outlet relative to the total molar amount of the compounds other than the starting material compound (e.g., chlorinated alkane etc.) in the gas flowing out from the reactor outlet.

Unlike the reaction of performing light irradiation using a mercury lamp of the prior art, the method for producing an alkane according to the present disclosure comprises performing a chlorination reaction by light irradiation using light having a wavelength in the visible light range, for example, performing the reaction in a liquid phase, to thereby increase the yield of the target compound.

### (1) Starting Material Compound

In the present disclosure, the starting material compound is an alkene represented by formula (2): 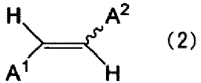 (wherein A¹ and A² are the same or different and represent a fluorine atom or a perfluoroalkyl group with the proviso that the case in which A¹ and A² both represent a fluorine atom is excluded).

In formula (2), A¹ and A² are the same or different and represent a fluorine atom or a perfluoroalkyl group, with the proviso that compounds wherein A¹ and A² both represent a fluorine atom are excluded from the compound of formula (2).

The perfluoroalkyl group represented by A¹ and A² is an alkyl group in which all of the hydrogen atoms are replaced with a fluorine atom. The perfluoroalkyl group has, for example, 1 to 20 carbon atoms, preferably 1 to 12 carbon atoms, more preferably 1 to 6 carbon atoms, still more preferably 1 to 4 carbon atoms, and particularly preferably 1 to 3 carbon atoms. The perfluoroalkyl group is preferably a linear or branched perfluoroalkyl group. The perfluoroalkyl group is preferably a trifluoromethyl group (CF₃-) or a pentafluoroethyl group (C₂F₅-).

The starting material compound alkene represented by formula (2) is preferably an alkene wherein A¹ and A² are the same or different and represent a fluorine atom or a trifluoromethyl group (CF₃-) from the standpoint of being able to produce a chlorinated alkane at a high conversion rate (yield) and with a high selectivity.

Examples of the alkene represented by formula (2), which is a starting material compound, include compounds represented by

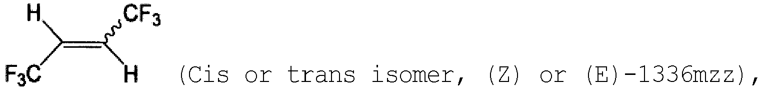

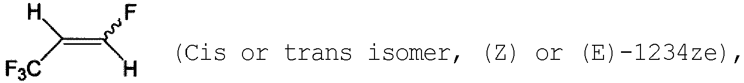

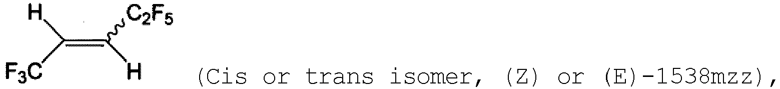

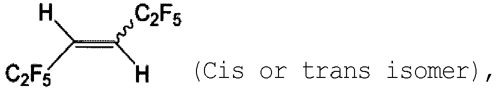

and the like.

These alkenes represented by formula (2) can be used alone or in a combination of two or more. Commercially available products can also be used as such alkenes.

### (2) Chlorination reaction

The chlorination reaction step in the present disclosure is performed by light irradiation using light having a wavelength in the visible light range.

In the chlorination reaction step in the present disclosure, for example, the starting material compound alkene represented by formula (2) is preferably a compound wherein A¹ and A² are the same or different and represent a fluorine atom or a trifluoromethyl group (CF₃-) from the standpoint of being able to produce a chlorinated alkane at a high conversion rate (yield) and with a high selectivity.

The chlorination reaction step in the present disclosure is a chlorination reaction according to the following reaction scheme.

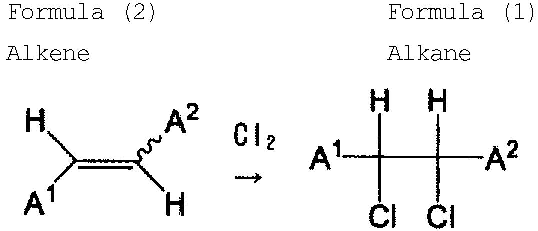

In formulas (1) and (2), A¹ and A² are the same or different and represent a fluorine atom or a perfluoroalkyl group with the proviso that the case in which A¹ and A² both represent a fluorine atom is excluded, and the compound of formula (2) represents a cis isomer and/or a trans isomer.

The chlorination reaction step in the present disclosure is preferably a chlorination reaction according to the following reaction scheme.

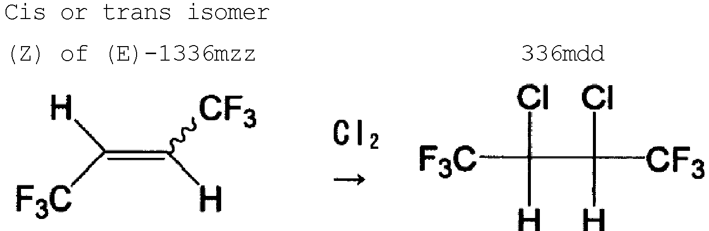

### Light irradiation

The chlorination reaction step in the present disclosure is performed by light irradiation using light having a wavelength in the visible light range. Preferably, the light irradiation in the chlorination reaction is performed by irradiation using light having a wavelength of 380 nm or more and 830 nm or less as a wavelength in the visible light range. There is no limitation on the light source used to perform the light irradiation as long as irradiation with light having a wavelength in the visible light range can be performed. Examples of preferable light sources include high-pressure mercury lamps, xenon lamps, fluorescent lamps, incandescent lamps, and the like.

### Liquid-phase reaction and solvent

The chlorination reaction in the present disclosure is preferably performed in a liquid phase.

In the chlorination reaction in the present disclosure, if the alkene represented by formula (2) used as a starting material compound is in the form of a liquid, no solvent needs to be added.

In the chlorination reaction in the present disclosure, a solvent can be used if necessary. The solvent can be water or a non-aqueous solvent. Preferred examples of non-aqueous solvents include chlorine-based solvents such as methylene chloride (dichloromethane), chloroform, trichloroethylene, and tetrachloroethylene. In the chlorination reaction in the present disclosure, the solvent is preferably at least one member selected from the group consisting of water and non-aqueous solvents.

In the chlorination reaction in the present disclosure, methylene chloride (dichloromethane) can be preferably used as a solvent because this solvent allows the chlorination reaction of an alkene used as a starting material compound to efficiently proceed even in a solvent and can produce a chlorinated alkane at a high conversion rate (yield) and with a high selectivity.

### Chlorination reaction temperature

In the chlorination reaction of an alkene in the present disclosure, there is no limitation on the temperature at which chlorine is added and light irradiation is performed using light having a wavelength in the visible light range.

In the chlorination reaction step in the present disclosure, for example, considering the fact that the (E)-1336mzz as an alkene has a boiling point of 7°C, the lower limit of the reaction temperature is usually -25°C, and preferably -15°C, from the viewpoint that the chlorination reaction can proceed more efficiently and the target compound can be obtained with a higher selectivity, and from the viewpoint of suppressing a decrease in the conversion rate.

In the chlorination reaction step in the present disclosure, the upper limit of the reaction temperature is usually 100°C, preferably 50°C, and more preferably 20°C, from the viewpoint that the chlorination reaction proceeds more efficiently and the target compound can be obtained with a higher selectivity, and from the viewpoint of suppressing a decrease in selectivity due to the decomposition or polymerization of the reaction product.

In the chlorination reaction step in the present disclosure, there is no limitation on the reactor as long as the chlorination reaction can be performed by light irradiation using light having a wavelength in the visible light range. A reaction in an open system (0 MPa) can also be used.

### Amount of Chlorine Used (Cl₂/Alkene Molar Ratio)

In the present disclosure, the amount of chlorine used is not particularly limited. In the present disclosure, from the standpoint of being able to efficiently subject an alkene to a chlorination reaction and produce a chlorinated alkane at a high conversion rate (yield) and with a high selectivity, the chlorine is preferably used in an amount of 0.1 to 10 moles (Cl₂/alkene molar ratio is 0.1 to 10), more preferably 1 to 5 moles (Cl₂/alkene molar ratio is 1 to 5), more preferably 1.5 to 3 moles (Cl₂/alkene molar ratio is 1.5 to 3), and particularly preferably 1.05 moles (Cl₂/alkene molar ratio is 1.05), per mole of the alkene.

### Closed Reaction System and/or Pressurized Reaction System

In the chlorination reaction in an alkene of the present disclosure, there is no limitation on the pressure applied when chlorine is added and light irradiation is performed using light having a wavelength in the visible light range.

If light irradiation can be performed in the chlorination reaction step in the present disclosure, the reaction can be performed in a closed reaction system and/or a pressurized reaction system. In the chlorination reaction in the present disclosure, as long as the reactor withstands the reaction, there is no limitation on the shape and structure of the reactor in which chlorine (Cl₂) is added to the starting material compound (alkene) and light irradiation is performed to allow the chlorination reaction to proceed.

The closed reaction system used in the chlorination reaction step in the present disclosure is preferably prepared by closing a reaction system using a batch-mode pressure-resistant reactor to perform a reaction. In the chlorination step in the present disclosure, the pressurized reaction system is preferably a reaction system in which the reaction pressure is 0 MPa or more. In present disclosure, the reaction pressure is a gauge pressure unless otherwise indicated.

As long as the closed reaction system and/or pressurized reaction system can perform the reaction by light irradiation using light having a wavelength in the visible light range, there is no limitation on the reactor. For example, it is preferred that the starting material compound, the solvent, etc. be placed in a pressure container such as an autoclave, heated to a proper reaction temperature with a heater, and allowed to react with stirring for a predetermined period of time. Examples of materials of the reactor include glass, stainless steel, iron, nickel, iron nickel alloy, and the like. The reaction is preferably performed in an atmosphere of an inert gas, such as nitrogen, helium, or carbon dioxide gas. For example, it is preferred that using a batch-mode pressure-resistant reactor (e.g., an autoclave), the reaction system is closed and the reaction is performed. Examples of materials of the reactor include glass, stainless steel, iron, nickel, iron nickel alloy, and the like.

In the chlorination reaction step in the present disclosure, the reaction pressure of the pressurized reaction system refers to a pressure inside the reactor used in the pressurized reaction system. In the chlorination reaction step in the present disclosure, the reaction is preferably performed at a reaction pressure of 0 MPa or more, more preferably 0.01 MPa or more, and still more preferably 0.02 MPa or more. In the chlorination reaction step in the present disclosure, the upper limit of the reaction pressure is usually around 0.1 MPa. The pressure in the reaction system can be increased by sending an inert gas, such as nitrogen, helium, or carbon dioxide gas, into the reaction system.

In the chlorination reaction step in the present disclosure, the reaction can be performed in a continuous, pressurized reaction mode, while taking out the liquid or taking out the reaction product formed into a gas by a method of, for example, using a continuous stirred-tank reactor (CSTR) to which a back pressure valve is attached.

In the present disclosure, after completion of the chlorination reaction, a purification treatment can be optionally performed in accordance with an ordinary method to obtain the chlorinated alkane represented by formula (1).

In the chlorination reaction step in the present disclosure, the reaction is performed in a closed reaction system and/or a pressurized reaction system to obtain the target compound at a higher selectivity and with a higher conversion rate.

### (3) Target Compound

The target compound in the present disclosure is an alkane (chlorinated alkane) represented by formula (1): 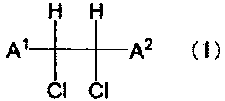 (wherein A¹ and A² are the same or different and represent a fluorine atom or a perfluoroalkyl group with the proviso that the case in which A¹ and A² both represent a fluorine atom is excluded).

In formula (1), A¹ and A² are the same or different and represent a fluorine atom or a perfluoroalkyl group.

The target compound alkane represented by formula (1) is preferably a compound wherein A¹ and A² are the same or different and represent a fluorine atom, or a trifluoromethyl group (CF₃-) .

Examples of the alkane represented by formula (1) to be produced include the following compounds represented by the following:

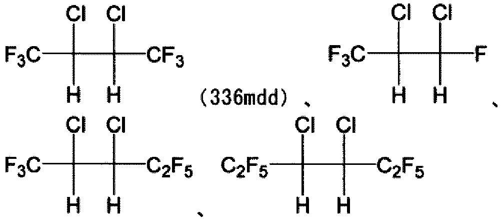

and the like.

In the method for producing an alkane according to the present disclosure, an alkene represented by formula (2) is used as a starting material compound; chlorine is added; and light irradiation is performed using light having a wavelength in the visible light range to allow a chlorination reaction to proceed, thus producing an alkane represented by formula (1).

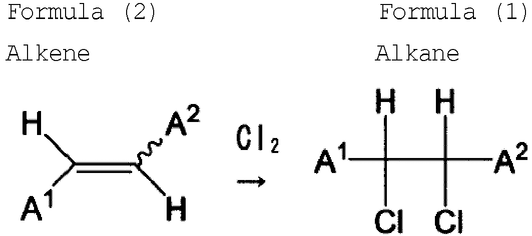

In formulae (1) and (2), A¹ and A² are the same or different and represent a fluorine atom or a perfluoroalkyl group, with the proviso that the case in which A¹ and A² both represent a fluorine atom is excluded; and the compound of formula (2) represents a cis isomer and/or a trans isomer.

In the chlorination reaction step in the present disclosure, the starting material compound alkene represented by formula (2) is preferably a compound wherein A¹ and A² are the same or different and represent a trifluoromethyl group (CF₃-). The chlorination reaction is preferably performed according to the following reaction scheme:

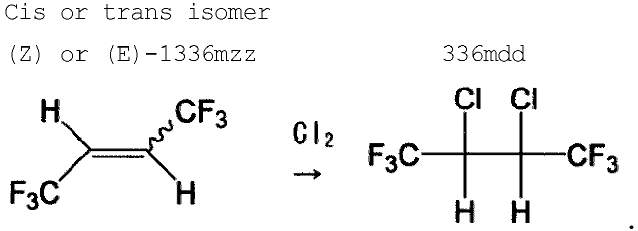

### (4) Composition Containing Alkane

The alkane represented by formula (1) (chlorinated alkane) can be obtained in the manner described above. However, as described above, the alkane represented by formula (1) may also be obtained in the form of a composition containing an alkane represented by formula (1) and an alkene represented by formula (2).

The alkane represented by formula (1) contained in the composition is preferably an alkane wherein A¹ and A² are the same or different and represent a fluorine atom or a trifluoromethyl group (CF₃-) .

In the composition containing an alkane represented by formula (1) of the present disclosure, the alkane represented by formula (1) is preferably present in an amount of 95 mol% or more, and more preferably 99 mol% or more, based on the total amount of the composition taken as 100 mol%. In the composition containing an alkane represented by formula (1) of the present disclosure, the alkane represented by formula (1) is preferably present in an amount of 80 mol% to 99.9 mol%, more preferably 90 mol% to 99.9 mol%, and still more preferably 95 mol% to 99.9 mol%, based on the total amount of the composition taken as 100 mol%.

The alkane composition represented by formula (1) of the present disclosure can contain an alkane represented by formula (1) and at least one additional compound comprising (selected from the group consisting of) a hydrochlorofluorocarbon (hydrochlorofluorocarbons) (HCFC) compound containing at least one chlorine (excluding the alkane represented by formula (1)).

The alkane represented by formula (1) is preferably present in an amount of 90 mol% or more and the additional compound is preferably present in an amount of 10 mol% or less, based on the total amount of the composition taken as 100 mol%.

The additional compound is preferably at least one selected from the group consisting of monochlorohexafluorobutane, trichlorohexafluorobutane, and tetrachlorohexafluorobutane.

In the method for producing the alkane according to the present disclosure, 2,2,3,3-tetrachloro-1,1,1,4,4,4-hexafluorobutane (HCFC-316maa, CF₃CCl₂CCl₂CF₃) can be produced in the chlorination reaction.

In the composition containing an alkane represented by formula (1) according to the present disclosure, for example, CF₃CHClCHClCF₃ (336mdd) is present in an amount of 99 mol% or more, and CF₃CCl₂CCl₂CF₃ (316maa) is present in an amount of 1 mol% or less, based on the total amount of the composition taken as 100 mol%.

The production method according to the present disclosure can produce an alkane represented by formula (1) (chlorinated alkane) with a particularly high selectivity, even when the alkane represented by formula (1) is obtained in the form of a composition containing the alkane represented by formula (1). Thus, it is possible to reduce the amount of components other than the alkane represented by formula (1) in the composition. The production method according to the present disclosure can reduce the labor for purification to obtain the alkane represented by formula (1).

The composition containing the alkane represented by formula (1) of the present disclosure is preferably used as an etching gas for forming state-of-the-art microstructures, such as semiconductors and liquid crystals, as well as refrigerants or heat transfer media, as in the case of the alkane represented by formula (1) alone. The composition containing the alkane represented by formula (1) of the present disclosure can also be effectively used for various applications, such as deposit gases, building blocks for organic synthesis, cleaning gases, etc. as well as etching gases.

The deposit gases refers to gases for depositing an etching-resistant polymer layer.

The building blocks for organic synthesis refer to a substance that can serve as a precursor of a compound that has a highly reactive skeleton. For example, when an alkane represented by formula (1) according to the present disclosure or the composition containing the alkane is reacted with a fluorine-containing organic silicon compound, such as CF₃Si(CH₃)₃, a fluoroalkyl group, such as CF₃ group, is introduced to convert the alkane into a substance that can be used as a cleaner or a fluorine-containing pharmaceutical intermediate.

The above describes embodiments of the present disclosure. However, various modifications of the embodiments and details can be made without departing from the spirit and scope of the claims.

### Examples

The following describes the present disclosure in more detail with reference to Examples. However, the present disclosure is not limited to these Examples.

### Examples: Reaction performed by irradiation of light having a wavelength in the visible light range

In the method for producing an alkane in Example 1, the compound used as a starting material was an alkene represented by formula (2) wherein A¹ and A² were a trifluoromethyl group (CF₃-) ; chlorine was added; and light irradiation was performed using light having a wavelength in the visible light range to allow a chlorination reaction to proceed according to the following reaction scheme:

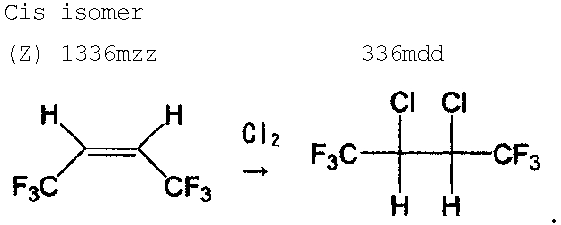

Starting material compound: (Z)1336mzz: (Z)-1,1,1,4,4,4-hexafluoro-2-butene Target compound: HCFC-336mdd: 2,3-dichloro-1,1,1,4,4,4-hexafluorobutane

The above chlorination reaction can also produce 2,2,3,3-tetrachloro-1,1,1,4,4,4-hexafluorobutane (HCFC-316maa, CF₃CCl₂CCl₂CF₃).

Methylene chloride (dichloromethane) as a solvent was placed in a glass reactor and (Z)1336mzz (starting material compound) was added thereto and a reaction was allowed to proceed at room temperature (10°C to 20°C) and normal pressure (0 MPa). A filter that excludes light with wavelength of 400 nm or less was attached to a fluorescent lamp and light having a wavelength in the visible light range was irradiated.

After stopping the reaction, the reaction mixture was cooled. Mass spectrometry was performed by gas chromatography (trade name: GC-2014, produced by Shimadzu Corporation) in accordance with gas chromatography-mass spectrometry (GC-MS), and structural analysis based on an NMR spectrum was performed using an NMR spectrometer (trade name: 400YH, produced by JEOL).

The results of mass spectrometry and structural analysis confirmed that 336mdd was obtained as a target compound. In Example 1, the conversion rate from (Z)1336mzz (starting material compound) was 99.1 mol%, the selectivity for 336mdd (target compound) was 98.95 mol%, and the selectivity for 316maa was 0.08 mol%.

In Example 2, the reaction was performed under the same conditions as in Example 1 except that no solvent was used. In Example 2, the conversion rate from (Z) 1336mzz (starting material compound) was 99.1 mol%, the selectivity for 336mdd (target compound) was 99.61 mol%, and the selectivity for 316maa was 0.17 mol%.

The results of the Examples show that when the target compound 2,3-dichloro-1,1,1,4,4,4-hexafluorobutane (HCFC-336mdd) is produced from the starting material compound (Z)-1,1,1,4,4,4-hexafluoro-2-butene ((Z)1336mzz), the alkene can be efficiently subjected to a chlorination reaction and the chlorinated alkane can be produced at a high conversion rate by adding chlorine and performing light irradiation using light having a wavelength in the visible light range.

### Comparative Examples: Reaction irradiated with light having a wavelength of 365 nm or 310 nm

The experiment method of the Examples was followed except that light irradiation was performed using light having a wavelength of 365 nm or 310 nm (UV wavelength), and the chlorination reaction, mass spectrometry, and structural analysis were performed in the same manner as in the Examples. The irradiation with light having a wavelength of 365 nm or 310 nm was performed by using a light reaction device (light source) produced by Techno-Sigma.

### Comparative Example: Reaction under light-shielded conditions

The experiment method of the Examples was followed except that the reaction was performed under light-shielded conditions. The chlorination reaction, mass spectrometry, and structural analysis were performed in the same manner as in the Examples. Using an autoclave, the chlorination reaction was allowed to proceed under light-shielded conditions.

Table 1 below shows the results of the Examples and Comparative Examples.

**Table 1**

| | Kind of light source | Reaction temperature (°C | Reaction pressure (MPa) | Amount of chlorine added Molar ratio (Cl₂/ (Z) 1336mzz) | Reaction time (after chlorine feeding) (hr) | Amount of (Z) 1336mzz Added (g) | Solvent CH2CI2 (g) | Conversion rate of the starting material compound (Z) 1336mzz (mol%) | Selectivity of the target compound 336mdd (mol%) | Selectivity of 316maa (mol%) | Other products (mol%) |
|---|---|---|---|---|---|---|---|---|---|---|---|
| Example 1 | Fluorescent lamp | 10 to 20 | 0 (normal pressure) | 1.05 | 4 | 25 | 25 | 99.1 | 98.95 | 0.08 | 0.97 |
| Example 2 | Fluorescent lamp | 10 to 20 | 0 (normal pressure) | 1.05 | 4 | 25 | 0 | 99.1 | 99.61 | 0.17 | 0.22 |
| | | | | | | | | | | | |
| Comp. Ex. 1 | 365nm | 10 to 20 | 0 (normal pressure) | 1.05 | 4 | 25 | 0 | 94.86 | 32.29 | 3.76 | 63.95 |
| Comp. Ex. 2 | 310nm | 10 to 20 | 0 (normal pressure) | 1.05 | 4 | 25 | 0 | 95.2 | 22.12 | 4.33 | 73.55 |
| Comp. Ex. 3 | Light - shielded Autoclave | 10 to 20 | 0.05 Autoclave | 1.05 | - | 25 | 0 | No reaction | - | - | - |

## Claims

1. A method for producing an alkane represented by formula (1) : 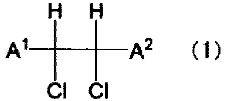 wherein A¹ and A² are the same or different and represent a fluorine atom or a perfluoroalkyl group with the proviso that the case in which A¹ and A² both represent a fluorine atom is excluded,
the method comprising the step of subjecting an alkene represented by formula (2): 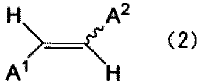
wherein A¹ and A² are as defined above,
to a chlorination reaction, and
the chlorination reaction step being performed by light irradiation using light having a wavelength in the visible light range.

2. The production method according to claim 1, wherein the chlorination reaction is performed in a liquid phase.

3. The production method according to claim 1 or 2, wherein the light irradiation is performed by irradiation using visible light having a wavelength of 380 nm or more and 830 nm or less.

4. A composition comprising
an alkane represented by formula (1): 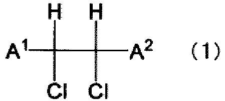 wherein A¹ and A² are the same or different and represent a fluorine atom or a perfluoroalkyl group with the proviso that the case in which A¹ and A² both represent a fluorine atom is excluded; and
at least one additional compound comprising a hydrochlorofluorocarbon (HCFC) compound containing at least one chlorine, excluding the alkane represented by formula (1).

5. The composition according to claim 4, wherein the alkane represented by formula (1) is present in an amount of 90 mol% or more and the additional compound is present in an amount of 10 mol% or less, based on the total amount of the composition taken as 100 mol%.

6. The composition according to claim 4 or 5, wherein the additional compound is at least one member selected from the group consisting of monochlorohexafluorobutane, trichlorohexafluorobutane, and tetrachlorohexafluorobutane.

7. The composition according to any one of claims 4 to 6, wherein the composition is for use as an etching gas, a refrigerant, or a heat transfer medium.
